# FASCICULE DE BREVET EUROPEEN

(11) **EP 1 708 631 B1**
(45) Date de publication et mention de la délivrance du brevet: **02.01.2008**
(21) Numéro de dépôt: 05717496.3
(22) Date de dépôt: 27.01.2005
(51) Int. Cl.: A61B 17/70

(54) **MATERIEL D OSTEOSYNTHESE VERTEBRALE**
WIRBEL-OSTEOSYNTHESE-VORRICHTUNG
VERTEBRAL OSTEOSYNTHESIS DEVICE

(30) Priorité: 27.01.2004 FR 0400743; 19.03.2004 US 554415 P
(43) Date de publication de la demande: 11.10.2006
(73) Titulaire: Medicrea International, 01700 Neyron (FR)
(72) Inventeur: SOURNAC, Denys, F-01600 REYRIEUX (FR); CAFFIERO, Jean-Philippe, F-69006 LYON (FR); CARLIER, François, F-44352 GUERANDE (FR)
(74) Mandataire: Jeannet, Olivier
(86) Numéro de dépôt international: PCT/FR2005/000173
(87) Numéro de publication internationale: WO 2005/082262

(56) Documents cités:
- EP-A- 0 582 857
- WO-A-01/60271
- FR-A- 2 735 011
- US-A- 5 242 446

## Description

La présente invention concerne un matériel d'ostéosynthèse vertébrale.

Un matériel d'ostéosynthèse vertébrale comprend généralement des organes d'ancrage osseux, tels que des vis pédiculaires, des pinces ou des crochets, une ou deux tiges de liaison, destinées à être reliées à ces organes d'ancrage et à être fixées aux vertèbres au moyen de ceux-ci, et des pièces de connexion de cette ou ces tiges de liaison à ces organes d'ancrage. Le matériel peut également comprendre des traverses réglables en longueur, qui relient transversalement deux tiges de liaison parallèles pour maintenir ces tiges l'une par rapport à l'autre.

Dans un type de matériel existant, chaque organe d'ancrage comporte un pion proximal fileté sur lequel peut être vissé un écrou, et chaque partie de connexion comprend une partie arrondie destinée à entourer une tige de liaison et deux ailes parallèles percées de trous. Ces ailes sont destinées à être engagées sur ledit pion proximal fileté et à être serrées, au moyen de cet écrou, contre une surface d'appui aménagée sur l'organe d'ancrage, ce serrage provoquant le serrage de ladite partie arrondie autour de la tige de liaison et assurant ainsi l'immobilisation longitudinale de cette tige par rapport à l'organe d'ancrage.

Dans un autre type de matériel existant, chaque organe d'ancrage comporte une partie de connexion en forme de "tulipe", présentant un logement dans lequel une tige de liaison peut être engagée.

Les organes d'ancrage peuvent être de type "polyaxial", c'est-à-dire permettant, avant serrage, une articulation du pion proximal fileté ou de la "tulipe" par rapport à la pièce de base de l'organe d'ancrage destinée à venir en prise avec l'os. Cette articulation facilite grandement l'assemblage des tiges de liaison aux organes d'ancrage.

Dans un organe d'ancrage "polyaxial" existant à pion proximal fileté, l'articulation est réalisée en aménageant une sphère à l'extrémité du pion proximal fileté et une cavité dans ladite pièce de base, cette cavité recevant la sphère et étant refermée en partie proximale pour retenir cette sphère.

Dans un organe d'ancrage "polyaxial" existant à "tulipe", ladite pièce de base comprend une sphère autour de laquelle vient s'articuler la partie de connexion en forme de "tulipe". Le document US 5 242 446 décrit un dispositif comprenant une vis engageable dans une vertèbre et un élément flexible allongé s'étendant à travers une ouverture de la vis et autour d'un tube de liaison pour connecter celui-ci avec la vertèbre.

Ces types d'articulation ont pour inconvénient de ne permettre qu'un débattement limité du pion proximal fileté ou de la "tulipe", ce qui peut rendre difficile dans certains cas la mise en place d'une tige sur des organes d'ancrage. De plus et surtout, les mouvements du patient conduisent à des mouvements du pion proximal fileté ou de la "tulipe" par rapport à la pièce de base, qui génèrent des frottements répétés de la sphère contre la paroi venant en appui contre cette sphère. Il en résulte un risque de diffusion non souhaitable de particules de métal dans l'organisme, et ce d'autant plus que les surfaces qui frottent les unes contre les autres sont relativement importantes.

Par ailleurs, les matériels d'ostéosynthèse vertébrale existants sont destinés à immobiliser deux vertèbres l'une par rapport à l'autre, pour éliminer tout mouvement relatif de ces vertèbres, ou pour rétablir la position adéquate d'une vertèbre par rapport à l'autre. Pour l'obtention de cette immobilisation, ces matériels sont conçus de façon à assurer un assemblage parfaitement rigide des tiges de liaison avec les organes d'ancrage.

Cet assemblage rigide peut cependant ne pas être souhaitable dans tous les cas. Il conduit en particulier à l'exercice de sollicitations importantes au niveau des zones osseuses d'ancrage desdits organes d'ancrage, ainsi qu'à des sollicitations augmentées au niveau des articulations vertébrales situées de part et d'autre du segment vertébral traité, qui peuvent conduire à des dégénérescences de ces articulations. En outre, il n'est pas adapté à des traitements d'affections non dégénératives, notamment au traitement de scolioses chez des patients jeunes.

La présente invention vise à remédier à l'ensemble de ces inconvénients.

Son objectif principal est donc de fournir un matériel d'ostéosynthèse vertébrale comprenant au moins un organe d'ancrage polyaxial, dans lequel le pion proximal fileté ou la "tulipe" de cet organe d'ancrage a un débattement important par rapport à la pièce de base de l'organe d'ancrage destinée à être fixée à l'os, et dans lequel le risque de diffusion de métal dans l'organisme est nettement réduit par rapport à un matériel existant.

Un autre objectif de l'invention est de fournir un matériel d'ostéosynthèse vertébrale permettant un assemblage non rigide, voire souple, des tiges de liaison avec les organes d'ancrage, avec un éventuel amortissement du mouvement des pièces mobiles.

Le matériel concerné comprend, de manière connue en soi, des organes d'ancrage osseux, tels que des vis pédiculaires, des pinces ou des crochets, une ou deux tiges de liaison, destinées à être reliées à ces organes d'ancrage et à être fixées aux vertèbres au moyen de ceux-ci, et des moyens de connexion de cette ou ces tiges à ces organes d'ancrage, au moins un de ces organes d'ancrage étant du type "polyaxial", c'est-à-dire comprenant une partie de connexion articulée par rapport à la pièce de base de l'organe d'ancrage destinée à être fixée à la vertèbre.

Selon l'invention, ladite partie de connexion et ladite pièce de base comprennent chacune un passage transversal et une partie transversale rigide de direction sensiblement perpendiculaire à la direction dudit passage, une partie transversale rigide de la partie de connexion ou la pièce de base étant engagée dans le passage transversal de la pièce de base ou la partie de connexion, et réciproquement, avec possibilité de pivotement de ces parties transversales rigides dans ces passages.

La partie de connexion articulée peut ainsi avoir un débattement très important, jusqu'à 180 degrés, alors que ce débattement n'excède pas 30 degrés dans un matériel existant. De plus et surtout, les deux parties transversales rigides ont des surfaces de contact mutuel réduites, limitant grandement les frottements au niveau de l'articulation. Cette limitation des frottements limite par conséquent dans une même mesure le risque de diffusion de particules métalliques dans l'organisme du patient.

Le terme "partie de connexion" doit être compris dans une acception très large : il peut en particulier s'agir d'un pion proximal fileté ou d'une partie de connexion en forme de "tulipe" tels que décrit plus haut ; il peut également s'agir d'une portion d'extrémité d'une tige de liaison.

Ledit passage transversal et ladite partie transversale rigide de la partie de connexion ou la pièce de base peuvent notamment être réalisés en aménageant un anneau, les deux anneaux de la partie de connexion et de la pièce de base étant engagés l'un dans l'autre à la manière de maillons d'une chaîne.

Ce passage transversal et cette partie transversale rigide peuvent également être aménagés sous forme de deux pattes latérales recevant un axe à travers elles, ces pattes et axe délimitant ledit passage et ledit axe formant ladite partie transversale rigide.

Lesdits passages transversaux et parties transversales rigides peuvent être ajustés l'un à l'autre de la même façon que des maillons de chaîne ; de préférence toutefois, chaque partie transversale rigide présente une surface de contact arrondie avec l'autre partie transversale rigide, le rayon de courbure de cette surface de contact étant supérieur au rayon de la section de l'autre partie transversale rigide.

Cette surface de contact arrondie rend ainsi possible un mouvement de roulement d'une partie transversale rigide par rapport à ladite surface de contact de l'autre partie transversale rigide, limitant encore les frottements d'une partie transversale rigide sur l'autre.

Les parties transversales rigides peuvent venir directement au contact l'une de l'autre, ou l'organe d'ancrage peut comporter une pièce intermédiaire, interposée entre ces parties transversales rigides. Dans le premier cas, les parties transversales rigides peuvent être en un matériau dur à faible coefficient de friction, ou peuvent comporter un revêtement, ou avoir subi un traitement, leur permettant d'avoir une dureté élevée et un faible coefficient de friction au niveau de leurs zones de contact réciproques. Dans le deuxième cas, ladite pièce intermédiaire peut être elle-même en un matériau à dureté élevée et à faible coefficient de friction.

Cette pièce intermédiaire peut notamment être conformée de manière à être retenue entre les deux parties transversales rigides grâce à la forme que présentent ces parties transversales rigides.

Selon un autre aspect de l'invention, ledit organe d'ancrage de type "polyaxial" comprend au moins une pièce ou une partie de pièce à structure élastiquement déformable, placée, après montage, entre ladite partie de connexion et ladite pièce de base, cette pièce ou partie de pièce à structure élastiquement déformable permettant une mobilité de la partie de connexion, et donc de la tige de liaison, par rapport à la pièce de base, avec amortissement.

Ainsi, dans le matériel selon l'invention, ladite partie de connexion n'est pas immobilisée par rapport à la pièce de base mais peut jouer vis-à-vis de celle-ci, afin d'autoriser des mouvements limités des vertèbres. Les sollicitations exercées par l'organe d'ancrage sur les zones osseuses d'ancrage sont ainsi notablement réduites, de même que les risques de sur-sollicitations au niveau des articulations vertébrales situées de part et d'autre du segment vertébral traité.

La pièce de base peut comprendre une pièce à structure élastiquement déformable et la partie de connexion peut comporter une autre pièce à structure élastiquement déformable, ces deux pièces venant porter l'une contre l'autre en position de montage.

Ladite partie de connexion peut comporter une surface courbe d'appui, propre à venir en appui contre une surface courbe d'appui correspondante de ladite pièce de base et à glisser contre cette surface lors des mouvements de ladite partie de connexion par rapport à ladite pièce de base. Notamment, ladite partie de connexion peut comporter une surface périphérique convexe, en forme de calotte sphérique, et ladite pièce de base peut comporter une surface périphérique concave correspondante.

L'invention sera bien comprise, et d'autres caractéristiques et avantages de celle-ci apparaîtront, en référence au dessin schématique annexé, représentant, à titre d'exemples non limitatifs, deux formes de réalisation possibles de pièces que comprend le matériel qu'elle concerne.
La figure 1 est une vue de côté, avant montage, de pièces formant une vis pédiculaire polyaxiale conforme à l'invention, selon une première forme de réalisation ; cette figure montre également une tige de liaison, un étrier de connexion en coupe transversale, et deux écrous permettant d'assembler une tige de liaison à cette vis ;
la figure 2 est une vue desdites pièces similaire à la figure 1 mais selon une direction perpendiculaire à la vue selon la figure 1 ;
la figure 3 est une vue des pièces montrées par la figure 1, après montage ;
la figure 4 est une vue de côté, avant montage, de deux pièces permettant de former une vis pédiculaire polyaxiale conforme à l'invention, selon la deuxième forme de réalisation ;
la figure 5 est une vue de côté de deux pièces permettant de former la vis pédiculaire polyaxiale, en cours de montage, et
la figure 6 est une vue de côté de trois pièces permettant de former la vis pédiculaire polyaxiale, après montage.

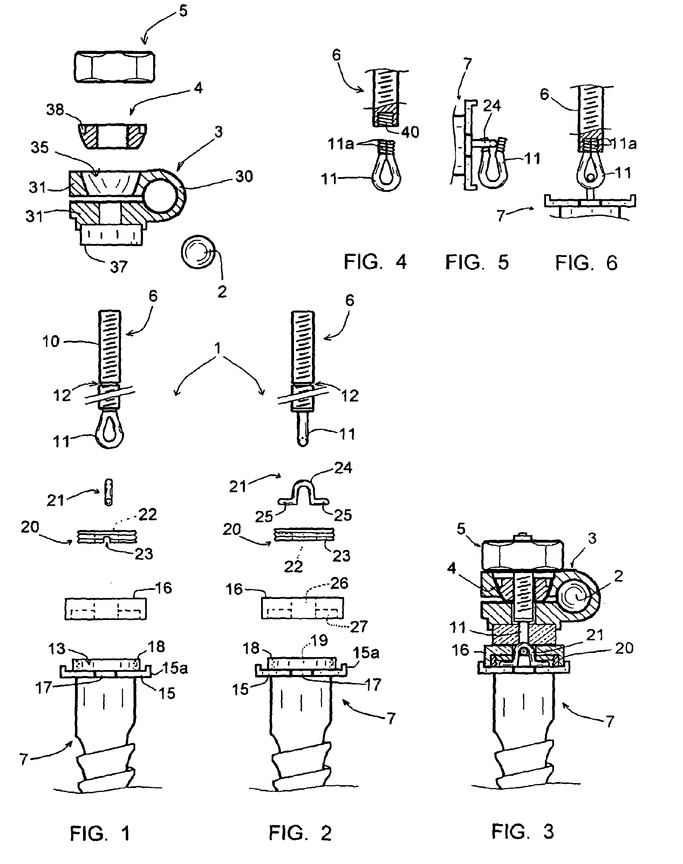

Les figures 1 et 2 représentent une vis pédiculaire polyaxiale 1, une tige 2 de liaison de plusieurs de ces vis 1, un étrier 3 de connexion de cette tige 2 à une de ces vis 1 et deux écrous 4, 5 permettant d'assembler la tige de liaison 2 à cette vis 1.

La vis 1 comprend un pion proximal fileté 6 et un corps de vis distal fileté 7. Le pion 6 est destiné à recevoir l'étrier 3 engagé sur lui et les écrous 4, 5 vissés sur lui tandis que le corps 7 est destiné à être inséré dans le pédicule d'une vertèbre.

Le pion 6 présente une partie cylindrique filetée 10, un anneau distal 11 et une zone 12 de diamètre réduit, permettant de casser sa portion proximale après mise en place et serrage de l'écrou 5, comme cela apparaît par comparaison des figures 1 et 3.

Le corps 7 comprend une collerette proximale 15, pourvue d'un rebord périphérique 15a, cette collerette 15 délimitant ainsi un logement de réception d'une rondelle 16 en matériau à structure élastiquement déformable, notamment en silicone ou en PMMA. Cette rondelle 16 permet, après montage, un amortissement du mouvement du pion 6 par rapport au corps 7, comme cela sera décrit plus loin.

La collerette 15 présente également plusieurs encoches radiales 17, notamment quatre encoches à 90° les unes des autres, permettant le maintien du corps 7 en rotation pendant le serrage des écrous 4 et 5.

Le corps 7 comprend en outre une paroi cylindrique proximale 18, délimitant un alésage taraudé 19.

La vis 1 comprend également une rondelle filetée 20 et une pièce en forme d'oméga 21.

La rondelle filetée 20 comprend un trou central 22 permettant le passage du pion 6 et de l'anneau 11, et une rainure diamétrale inférieure 23.

La pièce en forme d'oméga 21 comprend une partie centrale 24 pouvant être engagée dans l'anneau 11 et pouvant être engagée dans le trou 22 de la rondelle 20, et deux branches latérales 25 pouvant être reçues dans la rainure 23.

Comme cela se comprend, l'assemblage du pion 6 au corps 7 est réalisé en engageant la partie centrale 24 de la pièce 21 dans l'anneau 11, puis en engageant la rondelle 20 sur le pion 6 et l'anneau 11 jusqu'à introduction de ladite partie centrale 24 dans le trou 22 et desdites branches 25 dans la rainure 23, puis en vissant à bloc la rondelle 20 dans l'alésage taraudé 19. Pour son vissage, cette rondelle 20 comprend des cavités (non représentées) débouchant dans sa face proximale.

La rondelle 16 est ensuite mise en place sur la collerette 15, cette rondelle comprenant un trou central 26 permettant son engagement sur le pion 6 et l'anneau 11 et un embrèvement 27 recevant la paroi 18.

S'agissant des autres pièces montrées sur la figure 1, la tige de liaison 2 est cylindrique et présente une rigidité telle qu'elle permet le maintien de plusieurs vertèbres les unes par rapport aux autres. Cette tige 2 est toutefois déformable de manière à pouvoir être conformée en fonction de la correction du rachis à réaliser.

L'étrier 3 comprend une partie arrondie 30 destinée à entourer la tige de liaison 2 et deux ailes latérales parallèles 31 percées de trous pour l'engagement de l'étrier 3 sur le pion 6. Ces ailes 31 sont mutuellement distantes de telle sorte que, dans une position d'écartement, la tige 2 puisse être insérée et puisse coulisser dans la partie 30, et que, dans une position de rapprochement que leur confère le serrage de l'écrou 5, elles serrent la partie 30 autour de la tige 2, immobilisant cette dernière par rapport à l'étrier 3.

Comme le montre la figure 1, l'aile 31 proximale présente une cuvette proximale 35 de forme adaptée à la réception de l'écrou 4 sans appui axial, tandis que l'aile 31 distale comprend une paroi circulaire 36 formant un logement de réception d'une rondelle 37 à structure élastiquement déformable, notamment en silicone ou en PMMA, propre à coopérer avec la rondelle 16 pour amortir le mouvement du pion 6 par rapport au corps de vis 7.

L'écrou 4 comporte des encoches périphériques 38 pour sa manoeuvre en rotation. Il est dimensionné pour prendre appui uniquement contre la branche 31 distale de l'étrier 3 lorsqu'il est serré.

L'écrou 5 est dimensionné pour prendre appui contre la branche 31 proximale de l'étrier 3 lorsqu'il est serré.

En pratique, le nombre de vis 1 nécessaire au traitement à réaliser est mis en place dans les pédicules des vertèbres concernées, puis les étriers 3, avec la tige 2 engagée dans les parties 30, sont placés sur les pions 6.

L'écrou 4 est ensuite serré de manière contrôlée, par exemple au moyen d'un tournevis dynamométrique, pour réaliser un plus ou moins fort serrage des rondelles 16 et 37, selon le degré de l'amortissement recherché en fonction des caractéristiques du patient (état des disques intervertébraux, degré d'instabilité vertébrale, poids), puis l'écrou 5 est serré pour rapprocher les branches 31 et serrer ainsi la tige 2 dans la partie 30 de l'étrier 3.

Chaque étrier 3 peut comprendre une graduation gravée sur lui aux abords de la cavité 35 et l'écrou 4 peut comprendre un repère gravé sur lui, ce repère coopérant avec cette graduation pour permettre le serrage contrôlé de l'écrou 4.

Les portions proximales des pions 6 sont ensuite sectionnées.

Les figures 4 à 6 représentent une deuxième forme de réalisation de l'invention. Les pièces ou parties se retrouvant de manière identique ou similaire sont désignées par les mêmes références numériques.

Dans cette deuxième forme de réalisation, le corps de vis 7 comprend un anneau fixe 24, et l'anneau 11 se termine, du côté proximal, par deux branches 11a venant au contact l'une de l'autre. Les portions d'extrémité de ces branches 11a ont une forme hémi-cylindrique et sont filetées de manière à former, dans cette position de contact, un filet continu.

Le pion 6 comprend, quant à lui, un alésage taraudé 40 débouchant dans sa face distale.

Ainsi que le montre la figure 5, les deux branches 11a peuvent être écartées par déformation élastique de l'anneau 11 pour permettre l'engagement de l'anneau 24 à l'intérieur de l'anneau 11. Une fois cet engagement réalisé, les deux branches 11a viennent à nouveau en contact et l'anneau 11 peut être vissé et bloqué dans l'alésage 40.

Comme cela apparaît de ce qui précède, l'invention fournit un matériel d'ostéosynthèse vertébrale dans lequel le pion 6 a un débattement important par rapport au corps 7, et dans lequel le risque de diffusion de métal dans l'organisme est nettement réduit par rapport à un matériel existant, compte tenu des surfaces de contact réduites qu'ont les anneaux 11 et 24.

L'invention fournit également un matériel d'ostéosynthèse vertébrale permettant un assemblage non rigide des tiges de liaison 2 avec les organes d'ancrage 1.

Il va de soi que l'invention n'est pas limitée à la forme de réalisation décrite ci-dessus à titre d'exemple mais qu'elle s'étend à toutes les formes de réalisations couvertes par les revendications ci-annexées.

En particulier, le terme "anneau" doit être compris dans une acception la plus large, comme étant une partie d'assemblage définissant une ouverture et une zone de jonction, la zone de jonction d'un anneau pouvant être engagée dans l'ouverture de l'autre anneau et réciproquement, de telle sorte que les zones de jonction des deux anneaux puissent prendre appui l'une contre l'autre et assembler ladite pièce de base à ladite partie de connexion (pion proximal fileté ou "tulipe").

Ainsi, l'on ne sortirait pas du cadre de l'invention :
- en réalisant l'un des deux anneaux ouverts, pour permettre l'interpénétration des deux anneaux, puis en refermant l'anneau ouvert, notamment en réalisant un soudage de cet anneau à la pièce qui le comporte ;
- en réalisant l'un des deux anneaux ouverts, ou les deux anneaux ouverts ;
- en réalisant l'un des deux anneaux sous forme d'une chape recevant une goupille.

Le terme "partie de connexion articulée" doit également être compris dans une acception large : il peut s'agir d'un pion proximal fileté 6 tel que décrit plus haut, d'une partie de connexion en forme de "tulipe", présentant un logement dans lequel une tige de liaison 2 peut être engagée, ou encore de la portion d'extrémité d'une tige de liaison.

## Revendications

1. - Matériel d'ostéosynthèse vertébrale, comprenant des organes d'ancrage osseux, tels que des vis pédiculaires (1), des pinces ou des crochets, une ou deux tiges de liaison (2) destinées à être reliées à ces organes d'ancrage et à être fixées aux vertèbres au moyen de ceux-ci, et des moyens de connexion (6, 3) de cette ou ces tiges (2) à ces organes d'ancrage, au moins un de ces organes d'ancrage (1) étant du type "polyaxial", c'est-à-dire comprenant une partie de connexion (6) articulée par rapport à la pièce de base (7) de l'organe d'ancrage (1) destinée à être fixée à la vertèbre ;
matériel **caractérisé en ce que** ladite partie de connexion (6) et ladite pièce de base (7) comprennent chacune un passage transversal et une partie transversale rigide (11, 24) de direction sensiblement perpendiculaire à la direction dudit passage, ladite partie transversale rigide (11, 24) de la partie de connexion (6) ou de la pièce de base (7) étant engagée dans le passage transversal de la pièce de base (7) ou de la partie de connexion (6), et réciproquement, avec possibilité de pivotement de ces parties transversales rigides (11, 24) dans ces passages.

2. - Matériel selon la revendication 1, **caractérisé en ce que** ledit passage transversal et ladite partie transversale rigide (11, 24) de la partie de connexion (6) ou de la pièce de base (7) sont réalisés en aménageant un anneau (11) sur la partie de connexion (6) et un anneau (24) sur la pièce de base (7), les deux anneaux (11, 24) de la partie de connexion (6) ou de la pièce de base (7) étant engagés l'un dans l'autre à la manière de maillons d'une chaîne.

3. - Matériel selon la revendication 1 ou la revendication 2, **caractérisé en ce que** chaque partie transversale rigide (11, 24) présente une surface de contact arrondie avec l'autre partie transversale rigide (24, 11), le rayon de courbure de cette surface de contact étant supérieur au rayon de la section de l'autre partie transversale rigide (24, 11).

4. - Matériel selon la revendication 1 ou la revendication 2, **caractérisé en ce que** l'organe d'ancrage comporte une pièce intermédiaire, interposée entre lesdites parties transversales rigides (11, 24).

5. - Matériel selon la revendication 3 ou la revendication 4, **caractérisé en ce que** les parties transversales rigides sont en un matériau dur à faible coefficient de friction, ou comportent un revêtement, ou ont subi un traitement, leur permettant d'avoir une dureté élevée et un faible coefficient de friction au niveau de leurs zones de contact réciproques, ou **en ce que** ladite pièce intermédiaire est elle-même en un matériau à dureté élevée et à faible coefficient de friction.

6. - Matériel selon la revendication 4 ou la revendication 5, **caractérisé en ce que** ladite pièce intermédiaire est conformée de manière à être retenue entre les deux parties transversales rigides grâce à la forme que présentent ces parties transversales rigides.

7. - Matériel selon l'une des revendications 1 à 6, **caractérisé en ce que** ledit organe d'ancrage (1) de type "polyaxial" comprend au moins une pièce (16, 37) ou une partie de pièce à structure élastiquement déformable, placée, après montage, entre ladite partie de connexion (6, 3) et ladite pièce de base (7), cette pièce (16, 37) ou partie de pièce à structure élastiquement déformable permettant une mobilité de la partie de connexion (6, 3), et donc de la tige de liaison (2), par rapport à la pièce de base (7), avec amortissement.

8. - Matériel selon la revendication 7, **caractérisé en ce que** ladite pièce de base (7) comprend une pièce (16) à structure élastiquement déformable et ladite partie de connexion (3) comporte une autre pièce (37) à structure élastiquement déformable, ces deux pièces (16, 37) venant porter l'une contre l'autre en position de montage.

9. - Matériel selon l'une des revendications 1 à 6, **caractérisé en ce que** ladite partie de connexion comporte une surface courbe d'appui, propre à venir en appui contre une surface courbe d'appui correspondante de ladite pièce de base et à glisser contre cette surface lors des mouvements de ladite partie de connexion par rapport à ladite pièce de base.

10. - Matériel selon la revendication 9, **caractérisé en ce que** ladite partie de connexion comporte une surface périphérique convexe, en forme de calotte sphérique et ladite pièce de base comporte une surface périphérique concave correspondante.

## Claims

1. -Vertebral osteosynthesis material, comprising bone anchoring instruments, such as pedicular screws (1), clamps or hooks, one or two connecting rods (2) intended to be connected to these anchoring instruments and to be fixed to the vertebrae using these rods, and means (6, 3) for connecting this or these rod(s) (2) to these anchoring instruments, at least one of these anchoring instruments (1) being of the "polyaxial" type, meaning comprising a connection part (6) hinged relative to the base piece (7) of the anchoring instrument (1) intended to be fixed to the vertebra;
material **characterized in that** said connection part (6) and said base piece (7) each comprise a transverse passage and a stiff transverse part (11, 24) having a direction substantially perpendicular to the direction of said passage, said stiff transverse part (11, 24) of the connection part (6) or of the base piece (7) being engaged in the transverse passage of the base piece (7) or of the connection part (6) and reciprocally, with the possibility of rotating these stiff transverse parts (11, 24) in these passages.

2. - Material according to claim 1, **characterized in that** said transverse passage and said stiff transverse part (11, 24) of the connection part (6) or the base piece (7) are made by developing a ring (11) in the connection part (6) and a ring (24) on the base piece (7), the two rings (11, 24) of the connection part (6) or the base piece (7) being engaged one in the other like the links of a chain.

3. - Material according to claim 1 or claim 2, **characterized in that** each stiff transverse part (11, 24) has a rounded contact surface with the other stiff transverse part (24, 11), the arc radius of this contact surface being greater than the radius of the section of the other stiff transverse part (24, 11).

4. - Material according to claim 1 or claim 2, **characterized in that** the anchoring instrument includes an intermediate piece, inserted between said stiff transverse parts (11, 24).

5. - Material according to claim 3 or claim 4, **characterized in that** the stiff transverse parts are in a hard material with a low friction coefficient, or having a covering, or having undergone a treatment, granting them great hardness and a low friction coefficient at their mutual contact zones, or **in that** said intermediate piece itself is in a material with great hardness and a low friction coefficient.

6. - Material according to claim 4 or claim 5, **characterized in that** said intermediate piece is modeled so as to be held between the two stiff transverse parts thanks to the shape of these stiff transverse parts.

7. - Material according to one of claims 1 to 6, **characterized in that** said "polyaxial"-type anchoring instrument (1) comprises at least one piece (16, 37) or part of a piece having an elastically malleable structure, placed, after assembly, between said connection part (6, 3) and said base piece (7), this piece (16, 37) or part of a piece having an elastically malleable structure enabling mobility of the connection part (6, 3), and therefore of the connection rod (2), relative to the base piece (7), with shock absorption.

8. - Material according to claim 7, **characterized in that** said base piece (7) comprises a piece (16) having an elastically malleable structure and said connection part (3) includes another piece (37) having an elastically malleable structure, these two pieces (16, 37) bearing against each other in the assembled position.

9. - Material according to one of claims 1 to 6, **characterized in that** said connection part includes a curved support surface, intended to bear against the corresponding curved support surface of said base piece and to slide against this surface during movements of said connection part relative to said base piece.

10. - Material according to claim 9, **characterized in that** said connection part includes a convex peripheral surface, in the shape of a spherical cap and said base piece includes a corresponding concave peripheral surface.

## Patentansprüche

1. Material zur Wirbelosteosynthese, Knochenverankerungsorgane umfassend wie Pedikelschrauben (1), Klammern oder Haken, einen oder zwei Verbindungsstäbe (2), die dazu bestimmt sind, mit diesen Verankerungsorganen verbunden und an den Wirbeln mittels derselben befestigt zu werden, und Mittel zum Verbinden (6, 3) dieses Stabs oder dieser Stäbe (2) mit diesen Verankerungsorganen, wobei mindestens eines dieser Verankerungsorgane (1) vom Typ "polyaxial" ist, das heißt, einen in bezug auf das Basisteil (7) des Verankerungsorgans (1) beweglichen Verbindungsabschnitt (6) umfasst, der dazu bestimmt ist, am Wirbel befestigt zu werden;
**dadurch gekennzeichnet, dass** dieser Verbindungsabschnitt (6) und dieses Basisteil (7) jeweils einen Transversaldurchgang und einen starren Transversalabschnitt (11, 24) etwa senkrecht zur Richtung dieses Durchgangs umfassen, wobei der starre Transversalabschnitt (11, 24) des Verbindungsabschnitts (6) oder des Basisteils (7) in den Transversaldurchgang des Basisteils (7) oder des Verbindungsabschnitts (6) eingreift, und umgekehrt, mit der Möglichkeit des Schwenkens dieser starren Transversalabschnitte (11, 24) in diesen Durchgängen.

2. Material nach Anspruch 1, **dadurch gekennzeichnet, dass** der Transversaldurchgang und der starre Transversalabschnitt (11, 24) des Verbindungsabschnitts (6) oder des Basisteils (7) durch Ausbildung eines Rings (11) auf dem Verbindungsabschnitt (6) und eines Rings (24) auf dem Basisteil (7) ausgeführt sind, wobei die zwei Ringe (11, 24) des Verbindungsabschnitts (6) oder des Basisteils (7) in der Art von Kettengliedern ineinander greifen.

3. Material nach Anspruch 1 oder 2, **dadurch gekennzeichnet, dass** jeder starre Transversalabschnitt (11, 24) eine abgerundete Kontaktfläche mit dem anderen starren Transversalabschnitt (24, 11) aufweist, wobei der Biegeradius dieser Kontaktfläche größer ist als der Radius des Segments des anderen starren Transversalabschnitts (24, 11).

4. Material nach Anspruch 1 oder 2, **dadurch gekennzeichnet, dass** das Verankerungsorgan ein Zwischenteil zwischen den starren Transversalabschnitten (11, 24) umfasst.

5. Material nach Anspruch 3 oder 4, **dadurch gekennzeichnet, dass** die starren Transversalabschnitte aus einem harten Werkstoff mit einem geringen Reibungswert sind oder eine Beschichtung aufweisen oder einer Behandlung unterzogen wurden, die es ihnen ermöglichen, eine große Härte und einen geringen Reibungswert im Bereich ihrer gegenseitigen Kontaktzonen zu haben, oder dadurch, dass das Zwischenteil selbst aus einem Werkstoff mit großer Härte und geringem Reibungswert ist.

6. Material nach Anspruch 4 oder 5, **dadurch gekennzeichnet, dass** das Zwischenteil derart ausgebildet ist, dass es zwischen den zwei starren Transversalabschnitten durch die Form dieser starren Transversalabschnitte gehalten wird.

7. Material nach einem der Ansprüche 1 bis 6, **dadurch gekennzeichnet, dass** das Verankerungsorgan (1) "polyaxialen" Typs mindestens ein Teil (16, 37) oder einen Teil-Abschnitt mit elastisch deformierbarer Struktur umfasst, das bzw. der nach Montage zwischen dem Verbindungsabschnitt (3, 6) und dem Basisteil (7) platziert ist, wobei dieses Teil (16, 37) oder dieser Teil-Abschnitt mit elastisch deformierbarer Struktur eine Mobilität des Verbindungsabschnitts (6, 3) und damit des Verbindungsstabs (2) in bezug auf das Basisteil (7) mit Dämpfung erlauben.

8. Material nach Anspruch 7, **dadurch gekennzeichnet, dass** das Basisteil (7) ein Teil (16) mit elastisch deformierbarer Struktur umfasst und der Verbindungsabschnitt (3) ein anderes Teil (37) mit elastisch deformierbarer Struktur aufweist, wobei sich diese zwei Teile (16, 37) in Montagestellung gegeneinander stützen.

9. Material nach einem der Ansprüche 1 bis 6, **dadurch gekennzeichnet, dass** der Verbindungsabschnitt eine gekrümmte Abstützfläche aufweist, die in der Lage ist, sich auf einer entsprechenden gekrümmten Abstützfläche des Basisteils abzustützen und bei Bewegungen des Verbindungsabschnitts in bezug auf das Basisteil gegen diese Fläche zu gleiten.

10. Material nach Anspruch 9, **dadurch gekennzeichnet, dass** der Verbindungsabschnitt eine periphere konvexe Fläche in Form einer kugelförmigen Kalotte aufweist und das Basisteil eine entsprechende periphere konkave Fläche aufweist.
